Europäisches Patentamt

European Patent Office

Office européen des brevets

(19)

(11) Veröffentlichungsnummer: **0 275 468**
**B1**

(12) **EUROPÄISCHE PATENTSCHRIFT**

(45) Veröffentlichungstag der Patentschrift: 06.02.91

(21) Anmeldenummer: 87118364.6

(22) Anmeldetag: 11.12.87

(51) Int. Cl.⁵: **A 61 K 31/66,** A 61 K 9/20, A 61 K 9/48

(54) Clodronat-haltige Arzneimittel und Verfahren zur Herstellung derselben.

(30) Priorität: 20.12.86 DE 3643758

(43) Veröffentlichungstag der Anmeldung:
27.07.88 Patentblatt 88/30

(45) Bekanntmachung des Hinweises auf die
Patenterteilung:
06.02.91 Patentblatt 91/06

(84) Benannte Vertragsstaaten:
AT BE CH DE ES FR GB GR IT LI LU NL SE

(56) Entgegenhaltungen:
EP-A-0 015 370    FR-A-2 005 229
DE-A-2 128 461
CHEMICAL ABSTRACTS, Band 100, Nr. 6, 6.
Februar 1984, Seite 570, Zusammenfassung
43398m, Columbus, Ohio, US; M. NARDELLI et
al.: "A structural study on metal binding of
gem-diphosphonates, bone growth regulators"

Die Akte enthält technische Angaben, die nach
dem Eingang der Anmeldung eingereicht
wurden und die nicht in dieser Patentschrift
enthalten sind.

(73) Patentinhaber: BOEHRINGER MANNHEIM
GMBH
Sandhofer Strasse 116
D-6800 Mannheim 31 (DE)

(72) Erfinder: Demmer, Fritz, Dr. rer. nat.
Kastanienweg 21
D-6945 Hirschberg-Leutershausen (DE)
Erfinder: Stemmle, Berthold, Dr. rer. nat.
Albweg 2
D-6832 Hockenheim (DE)

Courier Press, Leamington Spa, England.

EP 0 275 468 B1

# EP 0 275 468 B1

**Beschreibung**

Die Erfindung betrifft Clodronat-haltige Arzneimittel mit einem Wirkstoffgehalt von 80—95% und Verfahren zu ihrer Herstellung.

Natrium-Clodronat ist der chemische Kurzname für Methandichlordiphosphonsäure-dinatriumsalz. Es ist bekannt, daß diese Verbindung in Arzneimitteln zur Verhinderung der anomalen Mobilisierung und Ablagerung von Calciumphosphat in tierischen und menschlichen Geweben verwendet werden kann (vgl. DE—B 18 13 659). Da diese Verbindung in relativ hoher Dosierung über lange Zeit appliziert werden muß, um ihre Wirkung zu entfalten, wird zur bequemen Applikation eine orale Arzneimittelform mit hohem Wirkstoffgehalt, d.h. vergleichsweise geringer Tablettengröße, benötigt. Übliche Tablettenrezepturen, wie sie für ähnliche Stoffe beispielsweise in der vorstehenden DE—B 18 13 659 beschrieben sind, erwiesen sich überraschenderweise als unbrauchbar, da die entsprechenden Produkte aufgrund der Eigenschaften des Clodronats dazu neigen, entweder an den Matritzenwänden der automatischen Füllmaschinen zu kleben und damit eine einwandfreie Dosierung verhindern oder bei Kapselmaschinen an den Dosier- bzw. Ausstoßstempelchen selbst kleben bleiben, wobei sich sogar stärkere Beläge bilden können und die Dosierung wiederum ungenügend ist.

Es stellte sich daher die Aufgabe, eine Clodronat-Rezeptur zu entwickeln, die eine hohe Wirkstoffkonzentration einerseits mit einer guten Verarbeitbarkeit in automatischen Füllmaschinen andererseits vereinigt, ohne daß darunter die Auflösungsgeschwindigkeit der fertigen Präparate bzw. die Bioverfügbarkeit des Präparates leidet.

Diese Aufgabe wird dadurch gelöst, indem man Arzneimittel mit einem Gehalt von 80 bis ca. 95% Clodronat, 2 bis 10% Füllstoffen und 1 bis 10% Gleitmittel herstellt. Als besondere Ausführungsform für diese Arzneimittel kommen Tabletten oder Kapseln in Frage. Der Wirkstoffgehalt liegt in der Regel im Bereich von 400—1000 mg pro Einheitsdosis.

Das erfindungsgemäße Verfahren zur Herstellung von pharmazeutischen Zubereitungen mit einem Wirkstoffgehalt von Clodronsäure oder einem physiologisch verträglichen Salz davon besteht darin, daß man bezogen auf die fertige pharmazeutische Zubereitung einen Wirkstoffanteil von 80—95% mit einem Füllstoffanteil von 2—10% und einem Gleitmittelanteil von 0—5% trocken miteinander vermischt, mit einem wässrigen Bindemittel feucht granuliert und anschließend das Granulat trocknet, und dem Granulat zusätzlich ein Gleitmittel in einer Menge von 1—5% beimischt.

Die erfindungsgemäßen Arzneimittel enthalten Clodronat in einer Menge von 80—95% der Gesamtmitschung, bezogen auf die wasserfreie Form des Clodronats. Zur Verwendung kommt vorzugsweise das Dinatriumsalz, das in Form seines Tetrahydrates, aber auch in einer wasserfreien Form eingesetzt werden kann. Selbstverständlich können auch andere physiologisch verträgliche Salze, wie z.B. das Lithium-, Kalium-, Ammonium- oder Calciumsalz oder auch die freie Säure, gegebenenfalls in Verbindung mit einem geeigneten Puffer, eingesetzt werden. Während üblicherweise zur Erzielung einer raschen Wirkstoff-Freisetzung mit einem möglichst feinkörnigen, d.h. gemahlenen Wirkstoff gearbeitet wird, kann erfindungsgemäß ein ungemahlenes Produkt eingesetzt werden, welches auch Teilchen von über 400 μm enthält, wobei mindestens 70% eine Korngröße unter 400 μm und vorzugsweise von unter 200 μm aufweisen.

Die Rezeptur enthält weiterhin Füllstoffe in einer Menge von etwa 2—10%. Als solche können Stärke, Lactose, Glucose, Mannit, Calciumcarbonat, Calciumphosphat, Cellulose oder andere für diesen Zweck in der Technik bekannte Produkte verwendet werdem. Um die Auflösungsgeschwindigkeit in den gewünschten Freisetzungsbereich von 15—30 Minuten für eine in-vitro-Auflösungsrate von über 60 bzw. über 90% zu bringen, wird der Rezeptur ein Sprengmittel in einer Menge von 2—10% zugefügt. Als Sprengmittel haben sich inbesondere Carboxymethylstärke oder Carboxymethylcellulose sowie Kieselgel bewährt. Ein Austausch gegen entsprechend wirkende andere Produkte ist jedoch möglich. Die Rezeptur kann darüber hinaus noch ein Gleitmittel in einer Menge von 0—5% enthalten.

Erfindungsgemäß werden die vorstehenden Komponenten trocken gemischt und mit einem üblichen Bindemittel, wie Stärkekleister oder auch nur Wasser feucht granuliert. Das getrocknete Granulat sollte eine Korngröße von 0,1—2 mm, vorzugsweise etwa 1 mm, aufweisen. Zur weiteren Verarbeitung wird diesem Granulat nochmals ein Gleitmittel in einer Menge von 1—5% zugemischt, wobei Magnesiumstearat, Talkum, Paraffin, Aluminiumhydroxysilicat (Gleitol) oder ähnliche Produkte verwendet werden können. Besonders vorteilhaft ist eine Mischung aus Talkum und Magnesiumstearat, die in gleichen Teilen zugemischt werden. Es ist jedoch auch möglich, verschiedene Anteile dieser Mischung zu verwenden. Der Gesamtgehalt an Wirkstoff vermindert sich bei den so hergestellten Granulaten durch die nochmals zugesetzte Gleitmittelmenge nur geringfügig um ca. 1—2%. Der prozentuale Anteil an Gleitmitteln bei den fertigen Arzneitmitteln beträgt 1—10% und vorzugsweise 4—8%. Als besonders vorteilhaft hat sich der Bereich von 5—7% erwiesen.

Diese Mischung, die sich gut auf automatischen Anlagen abfüllen läßt, kann anschließend zu Tabletten verpreßt oder in übliche Gelatinekapseln abgefüllt werden. Tabletten bzw. Kapselgrößen werden vorzugsweise so gewählt, daß eine Wirkstoffkonzentration von 400—1000 mg pro Dosis erhalten wird. Es ist auch möglich, zunächst Pellets oder Minitabletten herzustellen und diese anschließend in Kapseln zu größeren Dosierungseinheiten, z.B. >500 mg Wirkstoff oder in Beutel oder Dosen abzufällen.

Die erfindungsgemäß zusammengesetzten Rezepturen lasen sich einwandfrei herstellen, ergeben gut

2

rieselfähige und abfüllbare Granulate, welche beim Verpressen oder Abfüllen in Kapseln nicht an den Werkzeugen haften. Es läßt sich so eine einwandfreie Dosierung der Einzeldosis mit einer Abweichung von weniger als 2%, normalerweise weniger als 1% vom Sollgewicht erreichen.

Wichtiges Kriterium für die Güte der erfindungsgemäßen Arzneimittel ist die Auflösungsrate der Einzeldosis. Bei der Bestimmung der Auflösungsrate (USP-paddle-method) wurde festgestellt, daß die erfindungsgemäßen Rezepturen bereits nach 15 Min. zu mehr als 60% und nach 30 Min. zu mehr als 90% gelöst sind, während eine Rezeptur mit einem Gleitmittelgehalt von etwa 8% eine Auflösungsrate von weniger als 30 bzw. weniger als 60% aufweist (vgl. Tabelle).

Die Herstellung der erfindungsgemäßen Arzneimittel sowie der Vergleichsprodukte ist in den folgenden Beispielen beschrieben, ohne daß dadurch die Erfindung beschränkt sein soll.

Beispiel 1
Standardrezeptur I

| | |
|---|---|
| Pos. 1 Natriumclodronat×4H$_2$O | 500 mg |
| Pos. 2 Talkum | 23 mg |
| Pos. 3 Maisstärke | 15 mg |
| Pos. 4 Natriumcarboxymethylstärke | 10 mg |
| Pos. 5 Magnesiumstearat | 2 mg |
| | 550 mg |

Standardrezeptur II

| | |
|---|---|
| Pos. 1 Natriumclodronat (wasserfrei) | 400 mg |
| Pos. 2 Talkum | 18 mg |
| Pos. 3 Maisstärke | 12 mg |
| Pos. 4 Natriumcarboxymethylstärke | 8 mg |
| Pos. 5 Magnesiumstearat | 2 mg |
| | 440 mg |

a) Ansatzgröße: 200 000 Stück
A. Pos. 1 und Pos. 2 mischen und mit einem 15 %-igen Kleister aus Pos. 3 und Wasser in einem Granulator (z.B. Diosna) granulieren, trocknen und sieben.
B. Pos. 4 und Pos. 5 mit Granulat aus A. mischen.
Das erhaltene Granulat ist gut rieselfähig, jedoch tritt nach kurzer Zeit ein Belag in den Dosierröhrchen und der Ausstoßvorrichtung der Abfüllmaschine auf, der eine einwandfreie Dosierung verhindert.

b) Ansatzgröße: 10 000 Stück
A. Pos. 1 bis Pos. 3 mischen und mit Wasser in einem Granulator (z.B. Diosna) granulieren, trocknen und sieben.
B. Pos. 4 und Pos. 5 mit Granulat A. mischen.
Rieselfähigkeit ungenügend, größere Dosierungsschwankungen sind unvermeidbar.

Beispiel 2
Erfindungsgemäße Rezeptur
Zu dem gemäß Beispiel 1a) hergestellten Granulat (Pos. 1—3) werden zusätzlich zu den Pos. 4 und Pos. 5
a) 2,5 mg Magnesiumstearat und 2,5 mg Talkum in einer Menge von ca. 1%
b) 5 mg Magnesiumstearat und 5 mg Talkum in einer Menge von ca. 2% bzw.
c) 10 mg Magnesiumstearat und 10 mg Talkum in einer Menge von ca. 4%
zugefügt.
Die unter a) und b) erhaltenen Granulate sind einwandfrei rieselfähig und in automatischen Abfüllmaschinen abfüllbar.
Durch weitere Erhöhung der Gleitmittelzusätze auf 4% (s.o. unter c), so daß sich zusammen mit dem bereits in der Rezeptur 1 enthaltenen Anteil von 4% eine Gesamtgleitmittelmenge von 8% ergibt, werden ebenfalls gut verarbeitbare Granulate erhalten, die jedoch in der fertigen Rezeptur eine ungenügende Auflösungsrate aufweisen (vgl. Tabelle).

# EP 0 275 468 B1

TABELLE

| Beispiel | Gleitmittel-zusatz | 15 Min. 30 Min. Auflösungsrate | | 15 Min. VK % Streuung | 30 Min. |
|---|---|---|---|---|---|
| 1a | — | — | — | — | — |
| 1b | — | — | — | — | — |
| 2a | 5 mg | 88 | 99 | 1,8 | 1,3 |
| 2b | 10 mg | 69 | 103 | 2,2 | 0 |
| 2c | 20 mg | 26 | 58 | 62,3 | 33,3 |

## Patentansprüche

1. Verfahren zur Herstellung von pharmazeutischen Zubereitungen mit einem Wirkstoffgehalt von Clodronsäure oder einem physiologisch verträglichen Salz davon, dadurch gekennzeichnet, daß man bezogen auf die fertige pharmazeutische Zubereitung einen Wirkstoffanteil von 80—95% mit einem Füllstoffanteil von 2—10% und einem Gleitmittelanteil von 0—5% trocken miteinander vermischt, mit einem wässrigen Bindemittel feucht granuliert und anschließend das Granulat trocknet, und dem Granulat zusätzlich ein Gleitmittel in einer Menge von 1—5% beimischt.

2. Verfahren gemäß Anspruch 1, dadurch gekennzeichnet, daß man dem Granulat ferner ein Sprengmittel von 2—10%, bezogen auf die fertige pharmazeutische Zubereitung beimischt.

3. Verfahren gemäß Anspruch 1 oder 2, dadurch gekennzeichnet, daß man ein wässriges Bindemittel mit einem Anteil von 2,5—20% Stärke verwendet.

4. Verfahren·gemäß einem der Ansprüche 1 bis 3, dadurch gekennzeichnet, daß man bezogen auf die fertige pharmazeutische Zubereitung einen Gleitmittelanteil von insgesamt 4—8%, insbesondere 5—7% verwendet.

5. Verfahren gemäß einem der Ansprüche 1 bis 4, dadurch gekennzeichnet, daß man als zusätzliches Gleitmittel Magnesiumstearat oder Talkum einsetzt.

6. Verfahren gemäß einem der Ansprüche 1 bis 5, dadurch gekennzeichnet, daß man den Wirkstoff in Form des Natriumsalzes, vorzugsweise in Form dessen Tetrahydrates, einsetzt.

7. Verfahren gemäß einem der Ansprüche 1 bis 6, dadurch gekennzeichnet, daß man das fertige Granulat anschließend zu Tabletten verpreßt oder in Kapseln abfüllt.

8. Pharmazeutische Zubereitung mit einem Wirkstoffgehalt von Clodronsäure oder einem physiologisch verträglichen Salz davon, dadurch gekennzeichnet, daß es einen Wirkstoffanteil von 80—95%, einen Füllstoffanteil von 2—10% und einen Gleitmittelanteil von 1—10% enthält und die Auflösungsrate des Wirkstoffes mindestens 60% nach 15 Minuten oder mindestens 90% nach 30 Minuten beträgt.

9. Pharmazeutische Zubereitung gemäß Anspruch 8, dadurch gekennzeichnet, daß bezogen auf die fertige pharmazeutische Zubereitung ein Sprengmittelanteil von 2—10% enthalten ist.

10. Pharmazeutische Zubereitung gemäß einem der Ansprüche 8 oder 9, dadurch gekennzeichnet, daß der Gleitmittelanteil 4—8% insbesondere 5—7% beträgt.

11. Pharmazeutische Zubereitung gemäß einem der Ansprüche 8 bis 10, dadurch gekennzeichnet, daß sie in einer zu Tabletten verpressten Form oder als eine in Kapseln abgefüllte Masse vorliegt und die Wirkstoffmenge pro Dosierungsform 400—1000 mg beträgt.

12. Pharmazeutische Zubereitung bestehend aus 500 mg Natriumclodronat×4H$_2$O, 15 mg Maisstärke, 23—28 mg Talkum, 10 mg Natriumcarboxymethylstärke und 2—7 mg Magensiumstearat.

13. Pharmazeutische Zubereitung gemäß einem der Ansprüche 8—12 zur Verhinderung der anomalen Mobilisierung oder Ablagerung von Kalziumphosphat in tierischem oder menschlichem Gewebe.

## Revendications

1. Procédé d'obtention de préparations pharmaceutiques ayant une teneur en substance active acide clodronique ou un de ses sels physiologiquement acceptables, caractérisé en ce qu'une proportion en substance active de 80—95%, une proportion en charge de 2—10% et une proportion en agent lubrifiant de 0—5%, par rapport à la préparation finie, sont mélangées entre elles, à sec, soumises à une granulation humide avec un liant aqueux, et ensuite, le granulé est séché, et mélangé encore avec un agent lubrifiant en une proportion de 1—5%.

2. Procédé selon la revendication 1, caractérisé en ce que l'on mélange le granulé en outre avec un agent de pulvérisation, en une proportion de 2—10%, par rapport à la préparation pharmaceutique finie.

4

3. Procédé selon la revendication 1 ou 2, caractérisé en ce que l'on utilise un liant aqueux ayant une teneur en amidon de 2,5—20%.

4. Procédé selon l'une quelconque des revendications 1 à 3, caractérisé en ce que l'on utilise une proportion d'agent lubrifiant de 4—8% au total, en paticulier 5—7%, par rapport à la préparation pharmaceutique finie.

5. Procédé selon l'une quelconque des revendications 1 à 4, caractérisé en ce que comme agent lubrifiant supplémentaire, on utilise du stéarate de magnésium ou du talc.

6. Procédé selon l'une quelconque des revendications 1 à 5, caractérisé en ce que l'on met en oeuvre la substance active sous la forme de son sel de sodium, de préférence sous la forme de son tétrahydrate.

7. Procédé selon l'une quelconque des revendications 1 à 6, caractérisé en ce que le granulé fini est ensuite compressé en comprimés ou conditionné dans des capsules.

8. Préparation pharmaceutique ayant une teneur en substance active acide clodronique ou un de ses sels physiologiquement acceptables, caractérisée en ce qu'elle contient une proportion en substance active de 80—95%, une proportion en charge de 2—10% et une proportion en agent lubrifiant de 1—10%, et que la vitesse de dissolution de la substance active est d'au moins 60% au bout de 15 minutes et d'au moins 90% au bout de 30 minutes.

9. Préparation pharmaceutique selon la revendication 8, caractérisée en ce que la teneur en agent de pulvérisation est de 2—10% par rapport à la préparation pharmaceutique finie.

10. Préparation pharmaceutique selon l'une des revendications 8 ou 9, caractérisée en ce que la teneur en agent lubrifiant est de 4—8%, en particuliar de 5—7%.

11. Préparation pharmaceutique selon l'une quelconque des revendications 8 à 10, caractérisée en ce qu'elle se présente sous une forme de comprimés ou sous la forme d'une masse conditionnée en capsules, et que la teneur en substance active est de 400—1000 mg par dose unitaire.

12. Préparation pharmaceutique constituée de 500 mg de clodronate de sodium×4H₂O; 15 mg d'amidon de maïs, 23—28 mg de talc, 10 mg d'amidon de carboxyméthylsodium et 2—7 mg de stéarate de magnésium.

13. Préparation pharmaceutique selon l'une quelconque des revendications 8—12, destinée à empécher la mobilisation ou le dépôt anormal de phosphate de calcium dans les tissus humains ou animaux.

## Claims

1. Process for the preparation of pharmaceutical compositions with an active material content of clodronic acid or of a physiologically acceptable salt thereof, characterised in that, referred to the final pharmaceutical composition, one dry mixes with one another a proportion of active material of 80—95% with a proportion of filling material of 2—10% and a proportion of lubricant of 0—5%, moist granulates with an aqueous binding agent and subsequently dries the granulate and additionally admixes the granulate with a lubricant in an amount of 1—5%.

2. Process according to claim 1, characterised in that one further admixes the granulate with a disintegration agent of 2—10%, referred to the final pharmaceutical composition.

3. Process according to claim 1 or 2, characterised in that one uses an aqueous binding agent with a proportion of 2.5—20% of starch.

4. Process according to one of claims 1 to 3, characterised in that, referred to the final pharmaceutical composition, one uses a proportion of lubricant of, in all, 4—8%, especially 5—7%.

5. Process according to one of claims 1 to 4, characterised in that, as additional lubricant, one uses magnesium stearate or talc.

6. Process according to one of claims 1 to 5, characterised in that one uses the active material in the form of the sodium salt, preferably in the form of its tetrahydrate.

7. Process according to one of claims 1 to 6, characterised in that one subsequently presses the final granulate into tablets or fills into capsules.

8. Pharmaceutical preparation with an active material content of clodronic acid or of a physiologically acceptable salt thereof, characterised in that it contains a proportion of active material of 80—95%, a proportion of filling material of 2—10% and a proportion of lubricant of 1—10% and the dissolving rate of the active material amounts to at least 60% after 15 minutes or at least 90% after 30 minutes.

9. Pharmaceutical composition according to claim 8, characterised in that, referred to the final pharmaceutical composition, it contains a proportion of disintegration agent of 2—10%.

10. Pharmaceutical composition according to claim 8 or 9, characterised in that the proportion of lubricant amounts to 4 to 8%, especially to 5—7%.

11. Pharmaceutical composition according to one of claims 8 to 10, characterised in that it is present in a form pressed into tablets or as a mass filled into capsules and the amount of active material per dosage form amounts to 400—1000 mg.

12. Pharmaceutical composition consisting of 500 mg. sodium clodronate×4H₄O, 15 mg. maize starch, 23—28 mg. talc, 10 mg. sodium carboxymethylstarch and 2—7 mg. magnesium stearate.

13. Pharmaceutical composition according to one of claims 8—12 for the prevention of anomalous mobilisation or deposition of calcium phosphate in animal or human tissue.